# EUROPEAN PATENT SPECIFICATION

(11) **EP 2 896 372 B1**
(45) Date of publication and mention of the grant of the patent: **09.05.2018**
(21) Application number: 12884676.3
(22) Date of filing: 11.09.2012
(51) Int. Cl.: A61B 8/12, A61B 5/00, A61B 5/026, A61B 8/00, A61B 8/08

(54) **IMAGE DIAGNOSIS DEVICE AND IMAGE PROCESSING METHOD**
BILDDIAGNOSEVORRICHTUNG UND BILDVERARBEITUNGSVERFAHREN
DISPOSITIF DE DIAGNOSTIC PAR IMAGERIE ET PROCÉDÉ DE TRAITEMENT D'IMAGE

(43) Date of publication of application: 22.07.2015
(73) Proprietor: Terumo Kabushiki Kaisha, Tokyo 151-0072 (JP)
(72) Inventor: KANEKO, Kenji, Ashigarakami-gun Kanagawa 259-0151 (JP)
(74) Representative: Casalonga
(86) International application number: PCT/JP2012/005749
(87) International publication number: WO 2014/041579

(56) References cited:
- WO-A1-2008/086613
- WO-A2-2008/057573
- JP-A- 2005 095 624
- JP-A- 2010 011 964
- US-A1- 2005 101 859
- US-A1- 2005 113 685
- US-A1- 2007 043 292

## Description

### Technical Field

The disclosure here generally relates to an imaging apparatus for diagnosis.

### Background Art

In the related art, an imaging apparatus for diagnosis has been used for diagnosis of arteriosclerosis, preoperative diagnosis in performing endovascular treatment using a high-performance catheter such as a balloon catheter or a stent, or for confirmation of postoperative results.

The imaging apparatus for diagnosis can include an intravascular ultrasound (IVUS) diagnosis apparatus and an optical coherence tomography (OCT) diagnosis apparatus, which respectively have different characteristics.

In addition, an imaging apparatus for diagnosis (imaging apparatus for diagnosis which includes an ultrasonic transceiver capable of transmitting and receiving ultrasonic waves and an optical transceiver capable of transmitting and receiving light) which has an IVUS function and an OCT function in combination has also been proposed (for example, refer to Patent Documents 1 and 2). According to these imaging apparatus for diagnosis, single scanning can generate both a cross-sectional image utilizing IVUS characteristics, which can enable measurement for a very deep region and a cross-sectional image utilizing OCT characteristics, which can enable high resolution measurement.

### Citation List

### Patent Literature

PTL 1: JP-A-11-56752
PTL 2: JP-T-2010-508973

Document WO-A1-2008/086613 represents the closest prior art disclosing the preamble of independent claim 1.

### Summary of Invention

The invention is defined by independent claim 1.

### Technical Problem

When a doctor performs diagnosis using these generated cross-sectional images, it can become important for the doctor to observe a specific range from the outside of a blood flow region to a blood vessel wall in the respective cross-sectional images. The reason can be that distribution, a size, and hardness of biological tissues in the range become important indexes in determining an intravascular state.

In accordance with an exemplary embodiment, for this reason, if this range can be accurately and quickly set as a region of interest (ROI) in the cross-sectional images, the doctor can not only quickly analyze the range upon diagnosis, but also can obtain an objective analysis result without depending on doctors' different skills.

In accordance with an exemplary embodiment, the present disclosure can enable a user to accurately and quickly set a region of interest in an imaging apparatus for diagnosis which can generate respective cross-sectional images by using a transceiver capable of transmitting and receiving ultrasonic waves and an optical transceiver capable of transmitting and receiving light.

### Solution to Problem

In order to achieve the above-described object, an imaging apparatus for diagnosis according to the present invention includes the following configurations.

That is, there is provided an imaging apparatus for diagnosis which has a transceiver in which an ultrasonic transceiver for transmitting and receiving ultrasonic waves and an optical transceiver for transmitting and receiving light are arranged, and which can generate an ultrasonic cross-sectional image and an optical cross-sectional image inside a blood vessel by transmitting the ultrasonic waves and the light while rotating the transceiver, and by using the reflected wave received by the ultrasonic transceiver and reflected from biological tissues and the reflected light received by the optical transceiver and reflected from the biological tissues while moving the transceiver into a blood vessel in an axial direction. The imaging apparatus for diagnosis can include a first generating means for generating a closed curve using the ultrasonic cross-sectional image at a predetermined position in the axial direction, a second generating means for generating a closed curve using the optical cross-sectional image at the predetermined position, and a setting means for setting a region between the closed curve generated by the first generating means and the closed curve generated by the second generating means as a region of interest in the ultrasonic cross-sectional image or the optical cross-sectional image, in a case where the ultrasonic cross-sectional image and the optical cross-sectional image are aligned and overlapped with each other at the predetermined position.

### Advantageous Effects of Invention

In accordance with an exemplary embodiment, a user can accurately and relatively quickly set a region of interest in an imaging apparatus for diagnosis, which can generate respective cross-sectional images by using an ultrasonic transceiver capable of transmitting and receiving ultrasonic waves and an optical transceiver capable of transmitting and receiving light.

Other characteristics and advantages of the disclosure will become apparent from the following description made with reference to the accompanying drawings. In the accompanying drawings, the same reference numerals are given to the same or similar configuration elements.

### Brief Description of Drawings

The accompanying drawings are incorporated in the description, configure a part of the description, represent embodiments of the imaging apparatus, and are used to describe principles of the imaging apparatus together with the description.
[Fig. 1] Fig. 1 is a view illustrating an external configuration of an imaging apparatus for diagnosis 100 according to an exemplary embodiment of the disclosure.
[Fig. 2] Fig. 2 is a view illustrating an overall configuration of a probe unit and a cross-sectional configuration of a distal end portion.
[Fig. 3] Fig. 3 is a diagram illustrating a cross-sectional configuration of an imaging core and an arrangement of an ultrasonic transceiver and an optical transceiver.
[Fig. 4] Fig. 4 is a diagram illustrating an exemplary functional configuration of the imaging apparatus for diagnosis 100.
[Fig. 5] Fig. 5 is a diagram illustrating an example of a user interface of the imaging apparatus for diagnosis 100.
[Fig. 6] Fig. 6 is a flowchart illustrating flow in an automatic setting process for an ROI in the imaging apparatus for diagnosis 100.
[Fig. 7] Fig. 7 is a diagram illustrating an example of the exemplary user interface of the imaging apparatus for diagnosis 100, and a diagram for illustrating a process of automatically setting the ROI.
[Fig. 8] Fig. 8 is a diagram illustrating an example of the exemplary user interface of the imaging apparatus for diagnosis 100, and a diagram illustrating a state where the ROI is automatically set.
[Fig. 9] Fig. 9 is a diagram illustrating an example of the exemplary user interface of the imaging apparatus for diagnosis 100, and a diagram illustrating an example of an analysis result in the ROI.
[Fig. 10] Fig. 10 is a diagram illustrating an example of the user interface of the imaging apparatus for diagnosis 100, and a diagram illustrating an example of an analysis result in the ROI.

### Description of Embodiments

Hereinafter, each exemplary embodiment will be described in detail with reference to the accompanying drawings. The embodiments described herein are only specific preferred examples, and thus various technically preferred limitations are imposed thereon. However, the scope of the present invention is not limited to these aspects of the embodiments unless otherwise described below in order to particularly limit the disclosure.

### [First Embodiment]

### <1. External Configuration of Imaging Apparatus for Diagnosis>

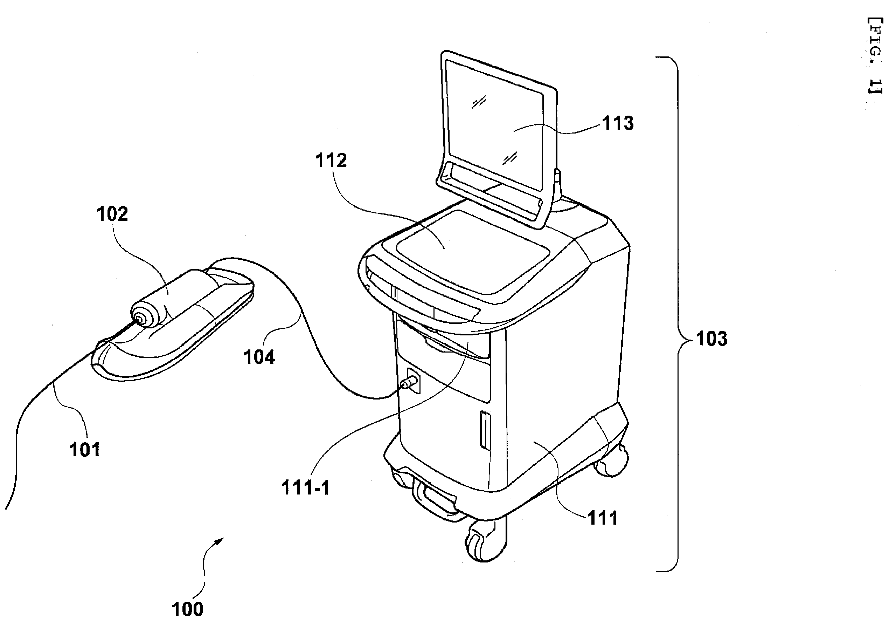

Fig. 1 is a view illustrating an external configuration of an imaging apparatus for diagnosis (imaging apparatus for diagnosis which includes an IVUS function and an OCT function) 100 according to an exemplary embodiment of the disclosure.

As illustrated in Fig. 1, the imaging apparatus for diagnosis 100 includes a probe unit 101, a scanner and pull-back unit 102, and an operation control device 103. The scanner and pull-back unit 102 and the operation control device 103 can be connected to each other by a signal line 104 so that various signals can be transmitted.

The probe unit 101 has an internally inserted imaging core including an ultrasonic transceiver which is directly inserted into a blood vessel, which transmits ultrasonic waves into the blood vessel based on a pulse signal, and which receives reflected waves from the inside of the blood vessel, and an optical transceiver which continuously transmits transmitted light (measurement light) into the blood vessel and which continuously receives reflected light from the inside of the blood vessel. The imaging apparatus for diagnosis 100 measures an intravascular state by using the imaging core.

The probe unit 101 is detachably attached to the scanner and pull-back unit 102. A motor incorporated in the scanner and pull-back unit 102 is driven, thereby regulating an intravascular operation in the axial direction and an intravascular operation in the rotation direction of the imaging core which is internally inserted into the probe unit 101. In addition, the scanner and pull-back unit 102 acquires the reflected wave received by the ultrasonic transceiver and the reflected light received by the optical transceiver, and transmits the reflected wave and the reflected light to the operation control device 103.

The operation control device 103 can include a function for inputting various setting values upon each measurement, and a function for processing data obtained by the measurement and for displaying an intravascular cross-sectional image (horizontal cross-sectional image and vertical cross-sectional image).

In the operation control device 103, the reference numeral 111 represents a main body control unit which generates ultrasonic data based on the reflected waves obtained by the measurement, and which generates an ultrasonic cross-sectional image by processing line data generated based on the ultrasonic data. Furthermore, the main body control unit 111 generates interference light data by causing the reflected light obtained by the measurement to interfere with reference light obtained by separating the light from a light source, and generates an optical cross-sectional image by processing the generated line data based on the interference light data.

The reference numeral 111-1 represents a printer and DVD recorder, which prints a processing result in the main body control unit 111 or stores the processing result as data. The reference numeral 112 represents an operation panel, and a user inputs various setting values and instructions via the operation panel 112. The reference numeral 113 represents an LCD monitor as a display device, which displays a cross-sectional image generated in the main body control unit 111.

### <2. Overall Configuration of Probe Unit and Cross-Sectional Configuration of Distal End Portion>

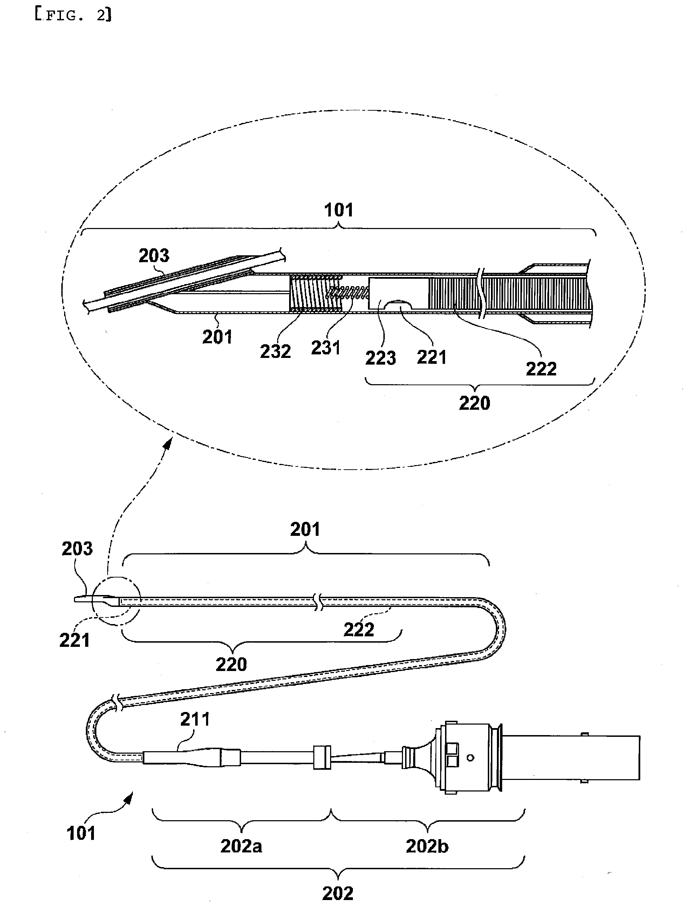

Next, an overall configuration of the probe unit 101 and a cross-sectional configuration of a distal end portion will be described with reference to Fig. 2. As illustrated in Fig. 2, the probe unit 101 is configured to include a long catheter sheath 201 to be inserted into the blood vessel and a connector unit 202 to be arranged on the front side of a user to be operated by the user without being inserted into the blood vessel. A guidewire lumen tube 203 configuring a guidewire lumen is disposed in the distal end of the catheter sheath 201. The catheter sheath 201 has a lumen which is continuously formed from a connection portion with the guidewire lumen tube 203 to a connection portion with the connector unit 202.

An imaging core 220 which internally includes a transceiver 221 in which the ultrasonic transceiver for transmitting and receiving the ultrasonic waves and the optical transceiver for transmitting and receiving the light are arranged, and which includes a coil-shaped drive shaft 222 internally including an electrical signal cable and an optical fiber cable and transmitting rotary drive power for rotating the transceiver 221 is inserted into the lumen of the catheter sheath 201 over substantially the entire length of the catheter sheath 201.

The connector unit 202 includes a sheath connector 202a configured to be integral with a proximal end of the catheter sheath 201, and a drive shaft connector 202b which is configured to rotatably fix the drive shaft 222 to the proximal end of the drive shaft 222.

An anti-kink protector 211 is disposed in a boundary section between the sheath connector 202a and the catheter sheath 201, which can help maintain predetermined rigidity, and can help prevent bending (kinking) caused by a rapid change in physical properties.

The proximal end of the drive shaft connector 202b is detachably attached to the scanner and pull-back unit 102.

Next, the cross-sectional configuration of the distal end portion of the probe unit 101 will be described. The imaging core 220 can include a housing 223 having the transceiver 221 in which the ultrasonic transceiver for transmitting and receiving the ultrasonic waves and the optical transceiver for transmitting and receiving the light are arranged, and can include the drive shaft 222 for transmitting the rotary drive power for rotating the housing 223 is inserted into the lumen of the catheter sheath 201 over substantially the entire length, thereby forming the probe unit 101.

The drive shaft 222 can cause the transceiver 221 to perform a rotary operation and an axial operation with respect to the catheter sheath 201, and has a property which can be flexible and can transmit rotation well. For example, the drive shaft 222 can be configured to have a multiplex and multilayer contact coil formed of a metal wire such as a stainless steel wire. Then, an electric signal cable and an optical fiber cable (optical fiber cable in a single mode) can be arranged inside the drive shaft 222.

The housing 223 can have a shape in which a short cylindrical metal pipe partially has a cutout portion, and can be formed by being cut out from a metallic ingot, or can be molded by means of metal powder injection molding (MIM). In addition, an elastic member 231 having a short coil shape can be disposed on the distal end side of the housing 223.

The elastic member 231 can be obtained by forming a stainless steel wire into a coil shape. The elastic member 231 is arranged on the distal end side, which can help prevent the imaging core 220 from being caught on the inside of the catheter sheath 201 when the imaging core 220 is moved forward and rearward.

The reference numeral 232 represents a reinforcement coil which is disposed in order to help prevent rapid bending of the distal end portion of the catheter sheath 201.

The guidewire lumen tube 203 has a guidewire lumen into which a guidewire can be inserted. The guidewire lumen tube 203 can be used in receiving the guidewire inserted into the blood vessel in advance and allowing the guidewire to guide the catheter sheath 201 to a lesion.

### <3. Cross-Sectional Configuration of Imaging Core>

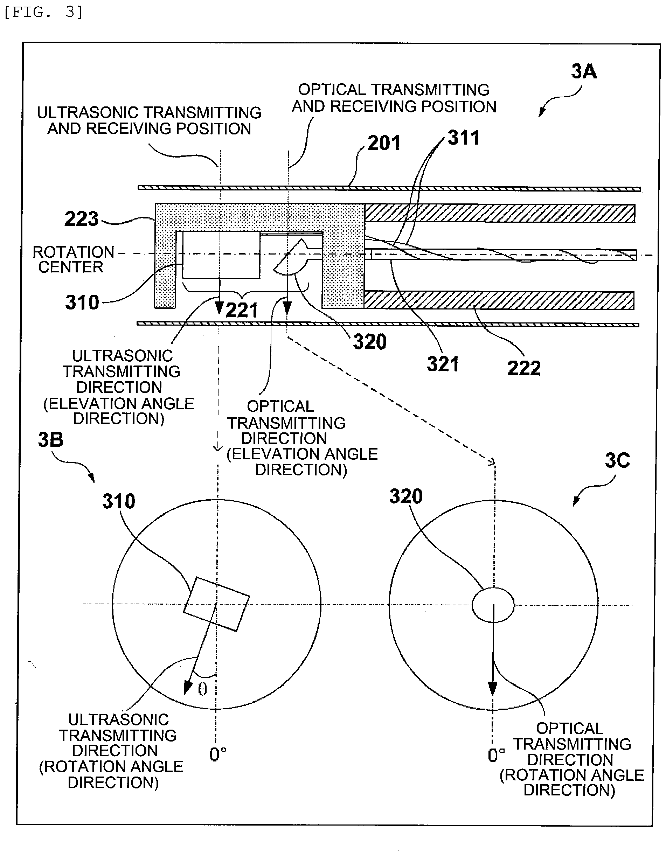

Next, a cross-sectional configuration of the imaging core 220 and an arrangement for the ultrasonic transceiver and the optical transceiver will be described. Fig. 3 is a diagram illustrating the cross-sectional configuration of the imaging core and the arrangement for the ultrasonic transceiver and the optical transceiver.

As illustrated in 3A of Fig. 3, the transceiver 221 arranged inside the housing 223 can include an ultrasonic transceiver 310 and an optical transceiver 320. The ultrasonic transceiver 310 and the optical transceiver 320 are respectively arranged along the axial direction on the rotation center axis (on the one-dot chain line in 3A) of the drive shaft 222.

In both of these, the ultrasonic transceiver 310 can be arranged on the distal end side of the probe unit 101, and the optical transceiver 320 can be arranged on the proximal end side of the probe unit 101.

In addition, the ultrasonic transceiver 310 and the optical transceiver 320 are attached inside the housing 223 so that an ultrasonic transmitting direction (elevation angle direction) of the ultrasonic transceiver 310 and a light transmitting direction (elevation angle direction) of the optical transceiver 320 are respectively approximately 90° with respect to the axial direction of the drive shaft 222. It is desirable to attach the ultrasonic transceiver 310 can be attached to the optical transceiver 320 by causing each transmitting direction to be slightly deviated from 90° so as not to receive the reflection on a surface inside the lumen of the catheter sheath 201.

An electric signal cable 311 connected to the ultrasonic transceiver 310 and an optical fiber cable 321 connected to the optical transceiver 320 are arranged inside the drive shaft 222. The electric signal cable 311 can be wound around the optical fiber cable 321 in a spiral shape.

3B of Fig. 3 is a cross-sectional view taken along a plane substantially orthogonal to the rotation center axis at an ultrasonic transmitting and receiving position. As illustrated in 3B of Fig. 3, when a downward direction from the paper surface is zero degrees, the ultrasonic transmitting and receiving direction (rotation angle direction (also referred to as an azimuth angle direction)) of the ultrasonic transceiver 310 is θ degrees.

3C of Fig. 3 is a cross-sectional view taken along a plane substantially orthogonal to the rotation center axis at an optical transmitting and receiving position. As illustrated in 3C of Fig. 3, when the downward direction from the paper surface is zero degrees, the light transmitting and receiving direction (rotation angle direction) of the optical transceiver 320 is zero degrees. That is, the ultrasonic transceiver 310 and the optical transceiver 320 are arranged so that the ultrasonic transmitting and receiving direction (rotation angle direction) of the ultrasonic transceiver 310 and the light transmitting and receiving direction (rotation angle direction) of the optical transceiver 320 are deviated from each other by θ degrees.

### <4. Functional Configuration of Imaging Apparatus for Diagnosis>

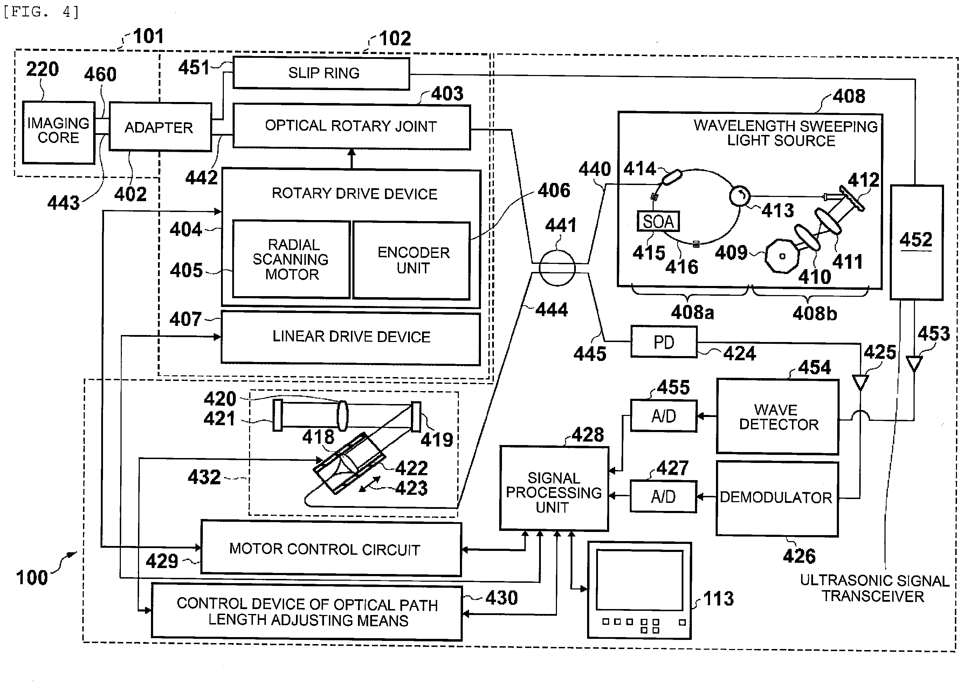

Next, a functional configuration of the imaging apparatus for diagnosis 100 will be described. Fig. 4 is a diagram illustrating the functional configuration of the imaging apparatus for diagnosis 100 which can include an IVUS function and an OCT function (here, a wavelength sweeping-type OCT as an example) in combination. An imaging apparatus for diagnosis including the IVUS function and other OCT functions in combination also has the same functional configuration. Therefore, description thereof will be omitted herein.

### (1) IVUS Function

The imaging core 220 can include the ultrasonic transceiver 310 inside the distal end of the image core 220. The ultrasonic transceiver 310 can transmit ultrasonic waves to biological tissues based on pulse waves transmitted by an ultrasonic signal transceiver 452, receives reflected waves (echoes)of the ultrasonic waves, and transmits the reflected waves to the ultrasonic signal transceiver 452 as an ultrasonic signal via an adapter 402 and a slip ring 451.

In the scanner and pull-back unit 102, a rotary drive portion side of the slip ring 451 is rotatably driven by a radial scanning motor 405 of a rotary drive device 404. In addition, a rotation angle of the radial scanning motor 405 is detected by an encoder unit 406. The scanning and pull-back unit 102 can include a linear drive device 407, and can regulate the axial operation of the imaging core 220 based on a signal from a signal processing unit 428.

The ultrasonic signal transceiver 452 can include a transmitting wave circuit and a receiving wave circuit (not illustrated). The transmitting wave circuit transmits the pulse waves to the ultrasonic transceiver 310 inside the imaging core 220 based on a control signal transmitted from the signal processing unit 428.

In addition, the receiving wave circuit receives an ultrasonic signal from the ultrasonic transceiver 310 inside the imaging core 220. The received ultrasonic signal can be amplified by an amplifier 453, and then is input to and detected by a wave detector 454.

Furthermore, an A/D converter 455 generates digital data (ultrasonic data) of one line by sampling the ultrasonic signal output from the wave detector 454, for example, at 30.6 MHz by an amount of 200 points. Although 30.6 MHz is used here, this is calculated on the assumption that the sampling of 200 points is performed for a depth of 5 mm when sound velocity is set to 1530 m/sec. Therefore, the sampling frequency is not particularly limited thereto.

The ultrasonic data in units of lines which is generated by the A/D converter 455 is input to the signal processing unit 428. The signal processing unit 428 converts the ultrasonic data into a gray scale, thereby generating an ultrasonic cross-sectional image at each position inside a blood vessel and outputting the ultrasonic cross-sectional image to an LCD monitor 113 at a predetermined frame rate.

The signal processing unit 428 is connected to a motor control circuit 429, and receives a video synchronization signal of the motor control circuit 429. The signal processing unit 428 generates the ultrasonic cross-sectional image in synchronization with the received video synchronization signal.

In addition, the video synchronization signal of the motor control circuit 429 is also transmitted to the rotary drive device 404, and the rotary drive device 404 outputs a drive signal synchronized with the video synchronization signal.

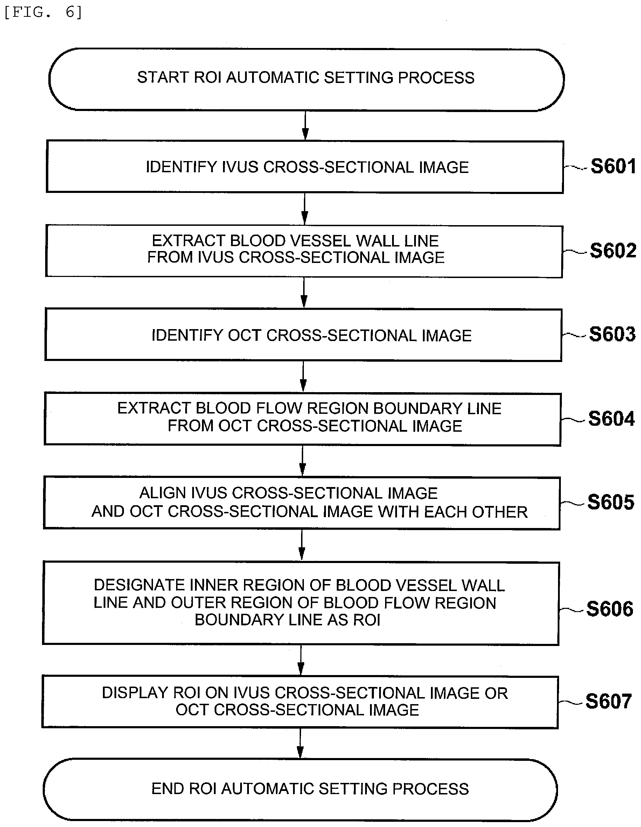

The above-described processing in the signal processing unit 428 and image processing relating to a user interface in the imaging apparatus for diagnosis 100 (to be described later with reference to Figs. 6 to 10) can be realized in such a way that a predetermined program causes a computer to execute the processing in the signal processing unit 428.

### (2) Function of Wavelength Sweeping-Type OCT

Next, a functional configuration of wavelength sweeping-type OCT will be described with reference to the same drawings. The reference numeral 408 represents a wavelength sweeping light source (swept laser), and is one type of an extended-cavity laser which can include an optical fiber 416 which is coupled to a semiconductor optical amplifier (SOA) 415 in a ring shape and a polygon scanning filter (408b).

Light output from the SOA 415 proceeds to the optical fiber 416, and enters the polygon scanning filter 408b. The light whose wavelength is selected here is amplified by the SOA 415, and is finally output from a coupler 414.

The polygon scanning filter 408b can select the wavelength in combination with a diffraction grating 412 for diffracting the light and a polygon mirror 409. Specifically, the light diffracted by the diffraction grating 412 can be concentrated on a surface of the polygon mirror 409 by two lenses (410 and 411). In this manner, only the light having a wavelength orthogonal to the polygon mirror 409 returns through the same optical path, and is output from the polygon scanning filter 408b. That is, time sweeping of the wavelength can be performed by rotating the polygon mirror 409.

For example, a 32-sided mirror can be used for the polygon mirror 409 whose rotation speed can be, for example, approximately 50000 rpm. A wavelength sweeping system in which the polygon mirror 409 and the diffraction grating 412 can be combined with each other, which can enable high speed and high output wavelength sweeping.

The light of a wavelength sweeping light source 408 which is output from the coupler 414 is incident on one end of a first single mode fiber 440, and is transmitted to the distal end side. The first single mode fiber 440 can be optically coupled to a second single mode fiber 445 and a third single mode fiber 444 in an optical coupler 441 located in the middle therebetween. Therefore, the light incident on the first single mode fiber 440 is transmitted by being split into a maximum of three optical paths by the optical coupler 441.

On the further distal end side than the optical coupler 441 of the first single mode fiber 440, an optical rotary joint (optical coupling unit) 403 which can transmit the light by coupling a non-rotating part (fixed portion) and a rotating part (rotary drive unit) to each other is disposed inside the rotary drive device 404.

Furthermore, a fifth single mode fiber 443 of the probe unit 101 can be detachably connected via the adapter 402 to a distal end side of a fourth single mode fiber 442 inside the optical rotary joint (optical coupling unit) 403. In this manner, the light from the wavelength sweeping light source 408 can be transmitted to the fifth single mode fiber 443 which can be inserted into the imaging core 220 and can be rotatably driven.

The transmitted light is emitted from the optical transceiver 320 of the imaging core 220 to the biological tissues inside the blood vessel while a rotary operation and an axial operation are performed. Then, the reflected light scattered on a surface or inside the biological tissues is partially captured by the optical transceiver 320 of the imaging core 220, and returns to the first single mode fiber 440 side through a rearward optical path. Furthermore, the light is partially transferred to the second single mode fiber 445 side by the optical coupler 441, and is emitted from one end of the second single mode fiber 445. Thereafter, the light is received by an optical detector (for example, a photodiode 424).

The rotary drive unit side of the optical rotary joint 403 can be rotatably driven by the radial scanning motor 405 of the rotary drive device 404.

On the other hand, an optical path length variable mechanism 432 for finely adjusting an optical path length of reference light can be disposed in the distal end opposite to the optical coupler 441 of the third single mode fiber 444.

In order for variations in the length of an individual probe unit 101 to be absorbed when the probe unit 101 is replaced and newly used, the optical path length variable mechanism 432 includes optical path length changing means for changing an optical path length corresponding to the variations in the length.

The third single mode fiber 444 and a collimating lens 418 can be disposed on a one-axis stage 422 which is movable in an optical axis direction thereof as illustrated by an arrow 423, thereby forming the optical path length changing means.

Specifically, the one-axis stage 422 functions as the optical path length changing means having a variable enough range of the optical path length to absorb the variations in the optical path length of the probe unit 101 when the probe unit 101 is replaced. Furthermore, the one-axis stage 422 can also include an adjusting means for adjusting an offset. For example, even when the distal end of the probe unit 101 is not in close contact with the surface of the biological tissues, the one-axis stage can finely change the optical path length. In this manner, the optical path length can be set in a state of interfering with the reflected light from the surface position of the biological tissues.

The optical path length is finely adjusted by the one-axis stage 422. The light reflected on a mirror 421 via a grating 419 and a lens 420 is mixed with the light obtained from the first single mode fiber 440 side by the optical coupler 441 disposed in the middle of the third single mode fiber 444, and then is received by the photodiode 424.

Interference light received by the photodiode 424 in this way can be photoelectrically converted, and can be input to a demodulator 426 after being amplified by the amplifier 425. The demodulator 426 performs demodulation processing for extracting only a signal portion of the interference light, and an output therefrom is input to the A/D converter 427 as an interference light signal.

The A/D converter 427 performs sampling on the interference light signal, for example, at 180 MHz by an amount of 2048 points, and generates digital data (interference light data) of one line. The reason for setting the sampling frequency to 180 MHz is on the assumption that approximately 90% of wavelength sweeping cycles (12.5 µsec) is extracted as the digital data of 2048 points, when a repetition frequency of the wavelength sweeping is set to 40 kHz. However, the sampling frequency is not particularly limited thereto.

The interference light data in the units of lines which is generated by the A/D converter 427 is input to the signal processing unit 428. The signal processing unit 428 generates data in a depth direction (line data) by performing frequency resolution on the interference light data using the fast Fourier transform (FFT), and the data is subjected to coordinate transformation. In this manner, an optical cross-sectional image is constructed at each intravascular position, and is output to the LCD monitor 113 at a predetermined frame rate.

The signal processing unit 428 is further connected to a control device of optical path length adjusting means 430. The signal processing unit 428 controls a position of the one-axis stage 422 via the control device of optical path length adjusting means 430.

The processing in the signal processing unit 428 can also be realized in such a way that a predetermined program causes a computer to execute the processing.

### <5. Description of User Interface>

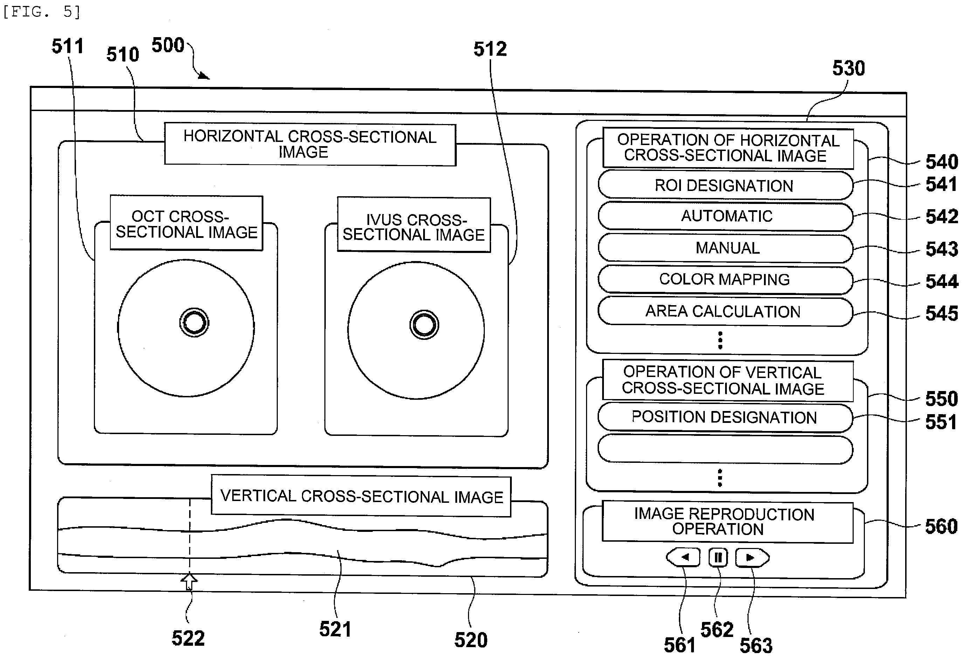

Next, a user interface displayed on the LCD monitor 113 will be described. Fig. 5 is a diagram illustrating an example of an examplary user interface 500 displayed on the LCD monitor 113.

As illustrated in Fig. 5, the user interface 500 can include a horizontal cross-sectional image display region 510 for displaying a horizontal cross-sectional image generated by the signal processing unit 428, a vertical cross-sectional image display region 520 for displaying a vertical cross-sectional image generated by the signal processing unit 428, and an operation region 530 for performing various operations on the horizontal cross-sectional image and the vertical cross-sectional image which are respectively displayed on the horizontal cross-sectional image display region 510 and the vertical cross-sectional image display region 520.

Furthermore, the horizontal cross-sectional image display region 510 can include an OCT cross-sectional image display region 511 for displaying an OCT cross-sectional image (optical cross-sectional image) generated by using an OCT function, and an IVUS cross-sectional image display region 512 for displaying an IVUS cross-sectional image (ultrasonic cross-sectional image) generated by using an IVUS function.

The vertical cross-sectional image display region 520 displays a vertical cross-sectional image 521 generated based on multiple IVUS cross-sectional images. A designator 522 displayed on the vertical cross-sectional image display region 520 is to designate a predetermined position in the axial direction of the vertical cross-sectional image 521. The OCT cross-sectional image and the IVUS cross-sectional image at the positions respectively designated by the designator 522 are displayed on the OCT cross-sectional image display region 511 and the IVUS cross-sectional image display region 512 which are described above.

The operation region 530 can include a horizontal cross-sectional image operation region 540 for operating the horizontal cross-sectional image inside the horizontal cross-sectional image display region 510, a vertical cross-sectional image operation region 550 for operating the vertical cross-sectional image inside the vertical cross-sectional image display region 520, and an image reproduction operation region 560 for continuously displaying (reproducing) the respective horizontal cross-sectional images inside the horizontal cross-sectional image display region 510.

An "ROI designation" button 541 for designating a region of interest (ROI) in the horizontal cross-sectional image (OCT cross-sectional image and/or IVUS cross-sectional image), an "automatic" button 542 and a "manual" button 543 which can be selected when the "ROI designation" button 541 is pressed down are arranged in the horizontal cross-sectional image operation region 540.

If the "automatic" button 542 is pressed down, a process for automatically setting the ROI (ROI automatic setting process) is performed by using the OCT cross-sectional image displayed on the OCT cross-sectional image display region 511 and the IVUS cross-sectional image displayed on the IVUS cross-sectional image display region 512. The ROI automatic setting process will be described in detail later.

On the other hand, if the "manual" button 543 is pressed down, a user can manually set the ROI on either the OCT cross-sectional image of the OCT cross-sectional image display region 511 or the IVUS cross-sectional image of the IVUS cross-sectional image display region 512 by using an operation device such as a mouse or a trackball on the operation panel 112.

A "color mapping" button 544 and an "area calculation" button 545 can be further arranged in the horizontal cross-sectional image operation region 540.

If the "color mapping" button 544 is pressed down, a color mapping process can be performed on biological tissues included in the designated ROI by using a color allocated in advance depending on the hardness of the biological tissues. The hardness of the biological tissues can be calculated by using strength and attenuation of the received signal. If the "area calculation" button 545 is pressed down, an area of the designated ROI can be calculated.

A "position designation" button 551 for displaying the designator 522 inside the vertical cross-sectional image display region 520 can be arranged in the vertical cross-sectional image operation region 550. The designator 522 can be displayed on the vertical cross-sectional image display region 520 by pressing down the "position designation" button 551. The horizontal cross-sectional image can be displayed at a desired position in the axial direction on the horizontal cross-sectional image display region 510 by using the operation device such as the mouse or the trackball on the operation panel 112 to move the designator 522 in the horizontal direction of the user interface 500.

A "rewind" button 561, a "stop" button 562, and a "reproduction" button 563 can be arranged in the image reproduction operation region 560. If the "rewind" button 561 is pressed down, the horizontal cross-sectional images displayed on the horizontal cross-sectional image display region 510 can be sequentially switched over to the horizontal cross-sectional images which are older in the generation sequence. That is, the horizontal cross-sectional images when proceeding in the direction opposite to the axial direction are continuously displayed. When the designator 522 is displayed on the vertical cross-sectional image display region 520, in synchronization with the switching of the horizontal cross-sectional images, the designator 522 is moved in the leftward direction of the user interface 500.

If the "reproduction" button 563 is pressed down, the horizontal cross-sectional images displayed on the horizontal cross-sectional image display region 510 are sequentially switched over to the horizontal cross-sectional images which are newer in the generation sequence. That is, the horizontal cross-sectional images when proceeding in the axial direction are continuously displayed. When the designator 522 is displayed on the vertical cross-sectional image display region 520, in synchronization with the switching of the horizontal cross-sectional images, the designator 522 is moved in the rightward direction of the user interface 500.

If the "stop" button 562 is pressed down, the switching of the horizontal cross-sectional images is stopped at the timing when pressed.

### <6. Flow of Automatic Setting Process for ROI>

Next, in the user interface 500, flow of the ROI automatic setting process which is performed by pressing down the "ROI designation" button 541 on the horizontal cross-sectional image operation region 540 and further pressing down the "automatic" button 542 will be described.

Fig. 6 is a flowchart illustrating the flow of the ROI automatic setting process in the imaging apparatus for diagnosis 100 according to an exemplary embodiment.

In Step S601, the IVUS cross-sectional image currently displayed on the IVUS cross-sectional image display region 512 is identified. In Step S602, a blood vessel wall line is extracted from the identified IVUS cross-sectional image. The blood vessel wall line is a closed curve obtained by extracting blood vessel wall positions from the IVUS cross-sectional image and connecting the positions as a continuous line segment. The blood vessel wall positions are extracted by using a known method.

For example, the known method for extracting the blood vessel wall positions can include a method for extracting the blood vessel wall positions, in which positions where a value of each pixel data arrayed in the line direction increases steeply are respectively extracted as candidate points of the blood vessel wall positions with regard to each line data configuring the IVUS cross-sectional image, and then the candidate points where a deviation amount between the adjacent candidate points is equal to or greater than a predetermined value are excluded therefrom. The blood vessel wall line may generate the closed curve by connecting the extracted blood vessel wall positions using a straight line, or may generate the closed curve by calculating an approximate curve based on the extracted blood vessel wall positions.

In Step S603, the OCT cross-sectional image currently displayed on the OCT cross-sectional image display region 511 is identified. In Step S604, a blood flow region boundary line is extracted from the identified OCT cross-sectional image. The blood flow region boundary line is a closed curve obtained by extracting boundary positions of a blood flow region from the OCT cross-sectional image and connecting the positions as a continuous line segment. The boundary positions of the blood flow region are extracted by using a known method.

For example, the known method for extracting the boundary positions of the blood flow region can include a method for extracting the boundary positions of the blood flow region, in which when a value of each pixel data arrayed in the line direction increases steeply and then decreases slowly, positions where the value increases steeply are respectively extracted as candidate points of the boundary positions of the blood flow region with regard to each line data configuring the OCT cross-sectional image, and then the candidate points where a deviation amount between the adjacent candidate points is equal to or greater than a predetermined value are excluded therefrom. The blood flow region boundary line may generate the closed curve by connecting the extracted boundary positions of the blood flow region using a straight line, or may generate the closed curve by calculating an approximate curve based on the extracted boundary positions of the blood flow region.

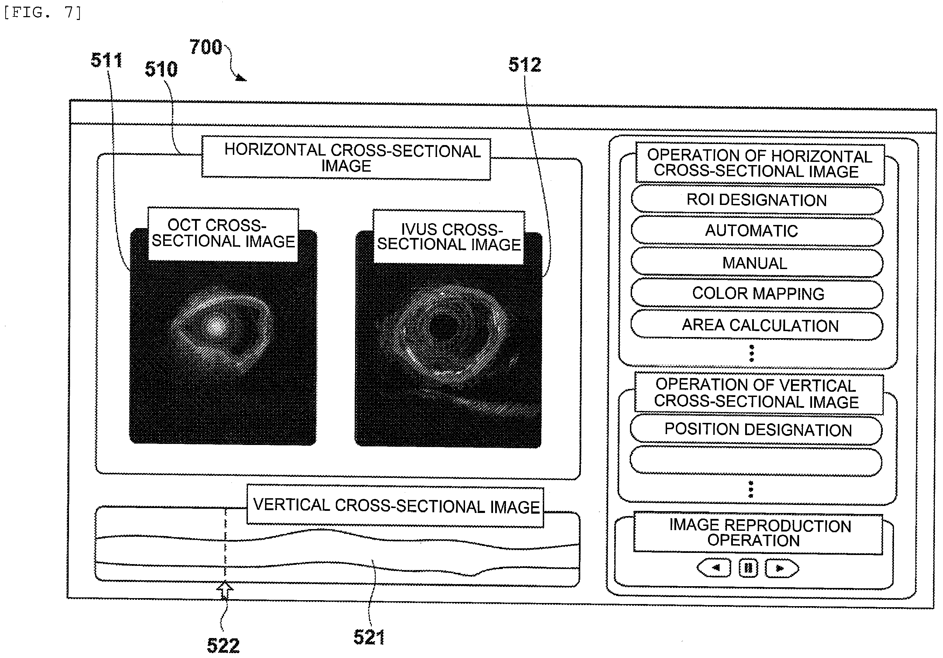

A user interface 700 in Fig. 7 indicates a state where the extracted blood vessel wall line is displayed so as to be identifiable by superimposing the blood vessel wall line on the IVUS cross-sectional image displayed on the IVUS cross-sectional image display region 512. In addition, the user interface 700 can indicate a state where the extracted blood flow region boundary line is displayed so as to be identifiable by superimposing the blood flow region boundary line on the OCT cross-sectional image displayed on the OCT cross-sectional image display region 511.

In Step S605, the IVUS cross-sectional image displayed on the IVUS cross-sectional image display region 512 and the OCT cross-sectional image displayed on the OCT cross-sectional image display region 511 are aligned with each other so that the image centers thereof are coincident with each other. In this manner, the blood vessel wall line and the blood flow region boundary line can be drawn on the same horizontal cross-sectional image (horizontal cross-sectional image of either the IVUS cross-sectional image obtained by extracting the blood vessel wall line or the OCT cross-sectional image obtained by extracting the blood flow region boundary line).

In Step S606, the ROI is set based on the blood vessel wall line and the blood flow region boundary line, which can be drawn on the same horizontal cross-sectional image. Specifically, an outer region of the blood flow region boundary line (region between the blood vessel wall line and the blood flow region boundary line) which is a region surrounded by the blood vessel wall line is set as the ROI.

In Step S607, the set ROI is displayed in a predetermined color so as to be identifiable by superimposing the ROI on the same horizontal cross-sectional image.

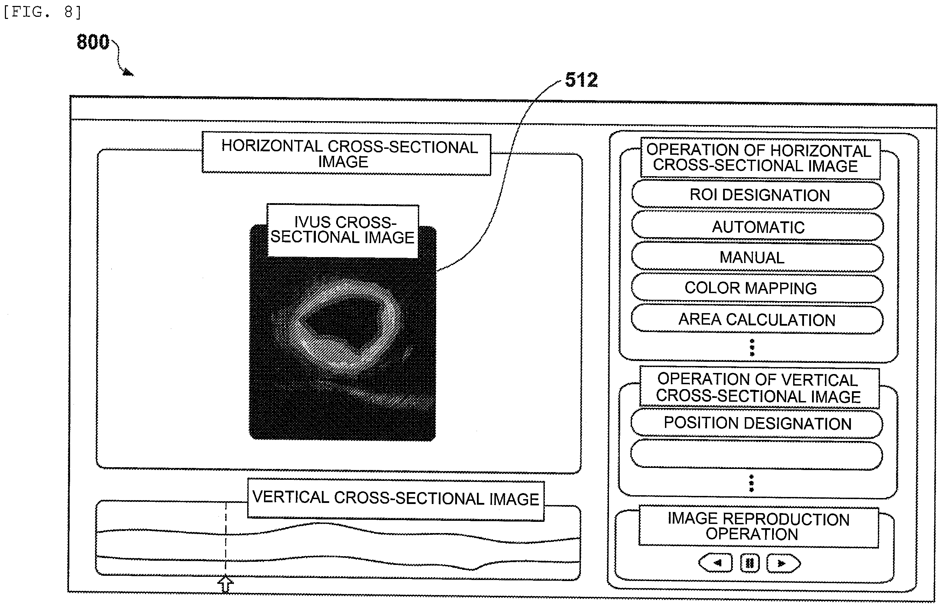

A user interface 800 in Fig. 8 can indicate a state where the blood vessel wall line, the blood flow region boundary line, and the ROI are displayed so as to be identifiable in the IVUS cross-sectional image.

As described above, the imaging apparatus for diagnosis 100 according to the present embodiment adopts a configuration in which the blood vessel wall line is extracted from the IVUS cross-sectional image by taking advantage of the IVUS characteristics which enable measurement for a very deep region, and in which the blood flow region boundary line is extracted from the OCT cross-sectional image by taking advantage of the OCT characteristics which enable high resolution measurement.

In this manner, it becomes possible to reliably and uniquely extract the blood vessel wall line and the blood flow region boundary line which are the boundary positions within the most important range during diagnosis of the cross-sectional image. In addition, the blood vessel wall line and the blood flow region boundary line which are respectively extracted from the IVUS cross-sectional image and the OCT cross-sectional image are drawn on the same horizontal cross-sectional image, and the region surrounded by the blood vessel wall line and the blood flow region boundary line is set as the ROI, thereby enabling a user to accurately and quickly set the ROI. As a result, when the user performs various analyses using the ROI, as compared to a case of manually setting the ROI, the user can obtain an objective analysis result without depending on the user's skill.

### <7. Analysis Example of ROI>

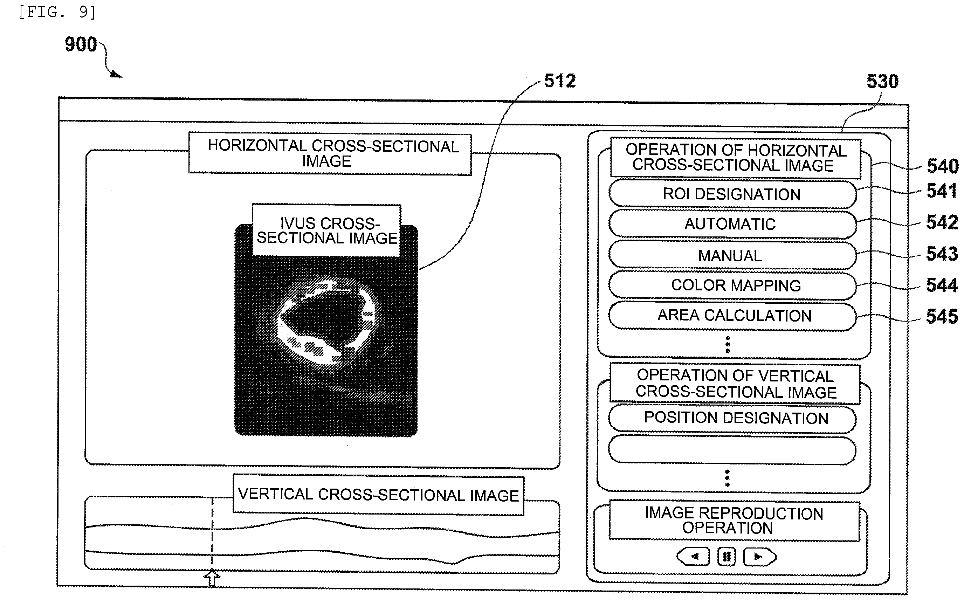

Next, an analysis example of the ROI set by the above-described ROI automatic setting process will be described. Fig. 9 illustrates a state where a color mapping process is performed on the ROI set by the ROI automatic setting process depending on the hardness of biological tissues.

If the "color mapping" button 544 of the horizontal cross-sectional image operation region 540 is pressed down in a state where the ROI is displayed (refer to Fig. 8), the IVUS cross-sectional image inside the ROI is analyzed, and the hardness of the respective biological tissues inside the ROI is calculated. As a method of calculating the hardness of the respective biological tissues based on the IVUS cross-sectional image, a known method is used.

A user interface 900 in Fig. 9 indicates a state where the color mapping process is performed on the ROI by using a color allocated in advance depending on the hardness of the respective biological tissues.

In the example illustrated in Fig. 9, a case of pressing down the "color mapping" button 544 has been described. However, for example, when an "area calculation" button 545 is pressed down, the IVUS cross-sectional image inside the ROI is analyzed, and an area inside the ROI is calculated.

As is apparent from the above description, the imaging apparatus for diagnosis 100 according to the present embodiment adopts a configuration in which the ROI is set by extracting the blood vessel wall line from the IVUS cross-sectional image, extracting the blood flow region boundary line from the OCT cross-sectional image, and combining both of these with each other.

As a result, it becomes possible to accurately and quickly set the ROI.

### [Second Embodiment]

The above-described first embodiment adopts a configuration of using the IVUS cross-sectional image when the blood vessel wall line and the blood flow region boundary line which are extracted are drawn on the same horizontal cross-sectional image. However, without being limited thereto, the disclose may be configured to use the OCT cross-sectional image, or may be configured to use the horizontal cross-sectional image obtained by synthesizing the IVUS cross-sectional image and the OCT cross-sectional image with each other. Alternatively, a configuration may be adopted so that the blood vessel wall line and the blood flow region boundary line are respectively drawn on the IVUS cross-sectional image and the OCT cross-sectional image. Alternatively, a configuration may be adopted so that a user can select whether the blood vessel wall line and the blood flow region boundary line are to be drawn on any horizontal cross-sectional images between the IVUS cross-sectional image and the OCT cross-sectional image.

In addition, the above-described first embodiment adopts a configuration of displaying the ROI (refer to Fig. 8) after the blood vessel wall line and the blood flow region boundary line (refer to Fig. 7) are temporarily displayed. However, without being limited thereto, the image apparatus for diagnosis may adopt a configuration of directly displaying the ROI (refer to Fig. 8) when the "automatic" button 541 is pressed down.

In addition, the above-described first embodiment adopts a configuration of automatically displaying the ROI (refer to Fig. 8) after the blood vessel wall line and the blood flow region boundary line (refer to Fig. 7) are displayed. However, without being limited thereto, the image apparatus for diagnosis may adopt a configuration of displaying the ROI, only when a user inputs a predetermined instruction after the user confirms whether the blood vessel wall line and the blood flow region boundary line (refer to Fig. 7) which are displayed are appropriate or not. Furthermore, the image apparatus for diagnosis may adopt a configuration so that correction can be manually added to the blood vessel wall line and the blood flow region boundary line (refer to Fig. 7) which are automatically extracted. In this case, a region surrounded by the blood vessel wall line and the blood flow region boundary line after the correction is made manually is set as the ROI.

### [Third Embodiment]

The above-described first embodiment adopts a configuration of displaying the color mapping inside the ROI when the color mapping is to be displayed, but the disclosure is not limited thereto.

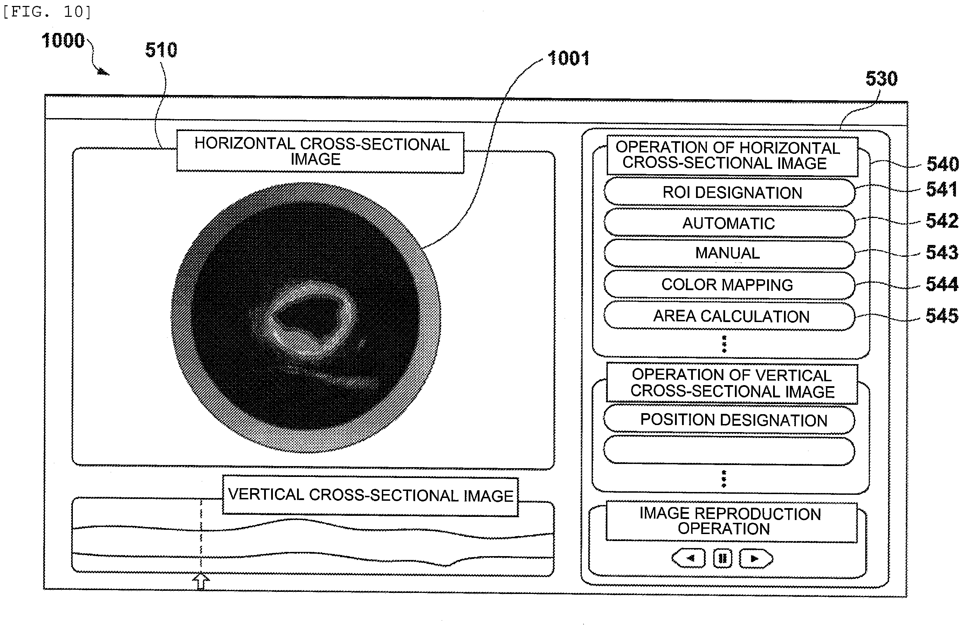

Fig. 10 is a diagram illustrating another display example in which the color mapping process is performed by pressing down the "color mapping" button 544. As illustrated in a user interface 1000 in Fig. 10, a configuration may be adopted in which an annular region 1001 having a predetermined width is displayed outside the ROI so that the color mapping inside the ROI is projected to and displayed on the annular region 1001. According to the color mapping, the hardness of the biological tissues inside the ROI can be relatively easily recognized and can be changed in the circumferential direction.

### [Another Embodiment]

Without being limited to the above-described embodiment, the present invention can be changed and modified in various ways without departing from the scope of the present invention. Therefore, in order to apprise the public of the scope of the present invention, the following Claims are appended herein.

## Claims

1. An imaging apparatus for diagnosis (100) which has a transceiver (221) in which an ultrasonic transceiver (310) for transmitting and receiving ultrasonic waves and an optical transceiver (320) for transmitting and receiving light are arranged, and which generates an ultrasonic cross-sectional image and an optical cross-sectional image inside a blood vessel by transmitting the ultrasonic waves and the light while rotating the transceiver (221), and by using the reflected wave received by the ultrasonic transceiver (310) and reflected from biological tissues and the reflected light received by the optical transceiver (320) and reflected from the biological tissues while moving the transceiver (221) into a blood vessel in an axial direction, the apparatus (100) comprising:
first generating means for generating a closed curve using the ultrasonic cross-sectional image at a predetermined position in the axial direction;
second generating means for generating a closed curve using the optical cross-sectional image at the predetermined position; and **characterized by**
the ultrasonic transceiver (310) for transmitting and receiving ultrasonic waves and the optical transceiver (320) for transmitting and receiving light being respectively arranged along the axial direction on the rotation center axis, and
setting means for setting the region between the closed curve generated by the first generating means and the closed curve generated by the second generating means as a region of interest in the ultrasonic cross-sectional image or the optical cross-sectional image, in a case where the ultrasonic cross-sectional image and the optical cross-sectional image are aligned and overlapped with each other at the predetermined position.

2. The imaging apparatus for diagnosis (100) according to Claim 1,
wherein the closed curve generated by the first generating means indicates a blood vessel wall, and the closed curve generated by the second generating means indicates a boundary of a blood flow region.

3. The imaging apparatus for diagnosis (100) according to Claim 2, further comprising:
display means for identifiably displaying the region of interest set by the setting means in the ultrasonic cross-sectional image or the optical cross-sectional image by using a predetermined color.

4. The imaging apparatus for diagnosis (100) according to Claim 3,
wherein the display means further identifiably displays the closed curve generated by the first generating means and the closed curve generated by the second generating means in the ultrasonic cross-sectional image or the optical cross-sectional image by using a predetermined color.

5. The imaging apparatus for diagnosis (100) according to Claim 1, further comprising:
processing means for processing the region of interest set by the setting means in the ultrasonic cross-sectional image or the optical cross-sectional image.

## Patentansprüche

1. Bildgebungsvorrichtung zur Diagnose (100), die einen Sendeempfänger (221) aufweist, in dem ein Ultraschall-Sendeempfänger (310) zum Senden und Empfangen von Ultraschallwellen und ein optischer Sendeempfänger (320) zum Senden und Empfangen von Licht angeordnet sind, und welche ein Ultraschallquerschnittsbild und ein optisches Querschnittsbild innerhalb eines Blutgefäßes durch Übertragen der Ultraschallwellen und des Lichts während des Rotierens des Sendeempfängers (221) und unter Verwendung der reflektierten Welle, die von dem Ultraschall-Sendeempfänger (310) empfangen und von biologischen Geweben reflektiert wurde, und dem reflektierten Licht, das von dem optischen Sendeempfänger (320) empfangen und von den biologischen Geweben reflektiert wurde, während der Sendeempfänger (221) in einer axialen Richtung in ein Blutgefäß bewegt wird, wobei die Vorrichtung (100) aufweist:
eine erste Erzeugungseinrichtung zum Erzeugen einer geschlossenen Kurve unter Verwendung des Ultraschall-Querschnittsbildes an einer vorbestimmten Position in der axialen Richtung;
eine zweite Erzeugungseinrichtung zum Erzeugen einer geschlossenen Kurve unter Verwendung des optischen Querschnittsbildes an der vorbestimmten Position; und **gekennzeichnet dadurch, dass**
der Ultraschall-Sendeempfänger (310) zum Senden und Empfangen von Ultraschallwellen und der optische Sendeempfänger (320) zum Senden und Empfangen von Licht, die jeweils entlang der axialen Richtung auf der Rotationsmittelachse angeordnet sind, und
Einstellmittel zum Einstellen des Bereichs zwischen der durch die erste Erzeugungseinrichtung erzeugten geschlossenen Kurve und der durch die zweite Erzeugungseinrichtung erzeugten geschlossenen Kurve als einen interessierenden Bereich in dem Ultraschallquerschnittsbild oder dem optischen Querschnittsbild, in einem Fall, wo das Ultraschallquerschnittsbild und das optische Querschnittsbild an der vorbestimmten Position ausgerichtet sind und einander überlappen.

2. Bildgebungsvorrichtung zur Diagnose (100) nach Anspruch 1,
wobei die von der ersten Erzeugungseinrichtung erzeugte geschlossene Kurve eine Blutgefäßwand anzeigt und die durch die zweite Erzeugungseinrichtung erzeugte geschlossene Kurve eine Grenze eines Blutströmungsbereichs anzeigt.

3. Bildgebungsvorrichtung zur Diagnose (100) nach Anspruch 2, ferner umfassend:
eine Anzeigeeinrichtung zum identifizierbaren Anzeigen des interessierenden Bereichs durch das Einstellmittel in dem Ultraschallquerschnittsbild oder dem optischen Querschnittsbild unter Verwendung einer vorbestimmten Farbe.

4. Bildgebungsvorrichtung zur Diagnose (100) nach Anspruch 3,
wobei die Anzeigeeinrichtung ferner die durch die erste Erzeugungseinrichtung erzeugte geschlossene Kurve und die durch die zweite Erzeugungseinrichtung erzeugte geschlossene Kurve in dem Ultraschallquerschnittsbild oder dem optischen Querschnittsbild unter Verwendung einer vorbestimmten Farbe identifizierbar anzeigt.

5. Bildgebungsvorrichtung zur Diagnose (100) nach Anspruch 1, ferner umfassend:
eine Verarbeitungseinrichtung zum Verarbeiten des interessierenden Bereichs, der durch die Einstelleinrichtung in dem Ultraschallquerschnittsbild oder dem optischen Querschnittsbild festgelegt ist.

## Revendications

1. Appareil d'imagerie diagnostique (100), comportant un émetteur-récepteur (221) dans lequel un émetteur-récepteur ultrasonore (310) destiné à émettre et à recevoir des ondes ultrasonores et un émetteur-récepteur optique (320) destiné à émettre et à recevoir de la lumière sont agencés, et générant une image de coupe ultrasonore et une image de coupe optique à l'intérieur d'un vaisseau sanguin par émission des ondes ultrasonores et de la lumière tout en faisant tourner l'émetteur-récepteur (221) et par utilisation de l'onde réfléchie reçue par l'émetteur-récepteur ultrasonore (310) et réfléchie par des tissus biologiques et de l'onde réfléchie reçue par l'émetteur-récepteur optique (320) et réfléchie par les tissus biologiques tout en déplaçant l'émetteur-récepteur (221) dans un vaisseau sanguin dans une direction axiale, l'appareil (100) comprenant :
un premier moyen de génération destiné à générer une courbe fermée en utilisant l'image de coupe ultrasonore à une position prédéterminée dans la direction axiale ;
un second moyen de génération destiné à générer une courbe fermée en utilisant l'image de coupe optique à la position prédéterminée ; et **caractérisé en ce que**
l'émetteur-récepteur ultrasonore (310) destiné à émettre et à recevoir des ondes ultrasonores et l'émetteur-récepteur optique (320) destiné à émettre et à recevoir de la lumière sont agencés respectivement le long de la direction axiale sur l'axe central de rotation, et
un moyen de définition sert à définir la région comprise entre la courbe fermée générée par le premier moyen de génération et la courbe fermée générée par le second moyen de génération comme région d'intérêt dans l'image de coupe ultrasonore ou l'image de coupe optique, dans le cas où l'image de coupe ultrasonore et l'image de coupe optique sont alignées et superposées l'une à l'autre à la position prédéterminée.

2. Appareil d'imagerie diagnostique (100) selon la revendication 1,
dans lequel la courbe fermée générée par le premier moyen de génération indique une paroi de vaisseau sanguin, et la courbe fermée générée par le second moyen de génération indique une frontière d'une région d'écoulement sanguin.

3. Appareil d'imagerie diagnostique (100) selon la revendication 2, comprenant en outre :
un moyen d'affichage destiné à afficher de manière identifiable la région d'intérêt définie par le moyen de définition dans l'image de coupe ultrasonore ou l'image de coupe optique par utilisation d'une couleur prédéterminée.

4. Appareil d'imagerie diagnostique (100) selon la revendication 3,
dans lequel le moyen d'affichage affiche en outre de manière identifiable la courbe fermée générée par le premier moyen de génération et la courbe fermée générée par le second moyen de génération dans l'image de coupe ultrasonore ou l'image de coupe optique par utilisation d'une couleur prédéterminée.

5. Appareil d'imagerie diagnostique (100) selon la revendication 1, comprenant en outre :
un moyen de traitement destiné à traiter la région d'intérêt définie par le moyen de définition dans l'image de coupe ultrasonore ou l'image de coupe optique.
